Europäisches Patentamt

(19) European Patent Office

Office européen des brevets

(11) **EP 0 865 289 B1**

(12) **FASCICULE DE BREVET EUROPEEN**

(45) Date de publication et mention
de la délivrance du brevet:
**13.02.2002 Bulletin 2002/07**

(21) Numéro de dépôt: **96941066.1**

(22) Date de dépôt: **25.11.1996**

(51) Int Cl.7: **A61K 35/78**, A61K 47/00

(86) Numéro de dépôt international:
**PCT/FR96/01860**

(87) Numéro de publication internationale:
**WO 97/20571 (12.06.1997 Gazette 1997/25)**

(54) **COMPOSITIONS A BASE D'ARCTIUM MAJUS**

ARZNEIZUBEREITUNGEN ENTHALTEND ARCTIUM MAJUS

ARCTIUM MAJUS COMPOSITIONS

(84) Etats contractants désignés:
**AT BE CH DE DK ES FR GR IT LI LU NL PT SE**

(30) Priorité: **04.12.1995 FR 9514292**

(43) Date de publication de la demande:
**23.09.1998 Bulletin 1998/39**

(73) Titulaire: **S.P.M.D, Societe Anonyme
80000 Amiens (FR)**

(72) Inventeur: **DEFRANCE, Pierre-Marie
F-80000 Amiens (FR)**

(74) Mandataire: **Sauvage, Renée
Cabinet Sauvage 65, Boulevard Soult
75012 Paris (FR)**

(56) Documents cités:
**EP-A- 0 524 873**

- **DATABASE WPI Week 9212 Derwent
  Publications Ltd., London, GB; AN 92-092922
  [12] XP002011970 A & JP 04 036 221 A (SUNSTAR
  KK,JP) 6 Février 1992**
- **DATABASE WPI Week 9013 Derwent
  Publications Ltd., London, GB; AN 90-096011
  [13] XP002011971 A & JP 02 048 514 A (SHISEIDO
  KK,JP) 19 Février 1990**
- **CHEMICAL ABSTRACTS, vol. 115, no. 20, 18
  Novembre 1991 Columbus, Ohio, US; abstract
  no. 214498, XP002011969 & MED.-BIOL. INF., vol.
  2, 1990, pages 28-31, I. IOCHKOVA ET AL.:
  "BURDOCK (ARCTIUM LAPPA):CHEMICAL
  COMPSNS. AND POSSIBLE APPLICATIONS IN
  COSMETICS"**

**Description**

La présente invention concerne une composition pharmaceutique à base d'Arctium majus, une solution lavante fabriquée à partir de celle-ci, et leur application au traitement du prurit.

Arctium majus est une plante médicinale bien connue, en particulier dans la médecine indigène traditionnelle, pour ses propriétés dépuratives, sudorifiques et antiseptiques, et dont on utilise essentiellement la racine fraîche, en particulier dans le traitement des maladies dermatologiques. Elle est habituellement administrée par voie interne, sous forme de décoction. Elle peut également être appliquée sous forme de topique, notamment dans le cas d'infections cutanées à staphylocoques. Dans ce dernier cas, elle est par exemple appliquée en compresses sur des furoncles ou en lotions, préparées à partir d'une décoction concentrée, sur une peau atteinte d'eczéma, d'acné, etc.

L'invention repose sur la découverte de l'effet anti-prurigineux d'Arctium majus.

Le prurit est un symptôme dermatologique courant qui se manifeste essentiellement par des démangeaisons au niveau de la peau, des muqueuses ou de la cornée. On distingue le prurit localisé (qui résulte souvent de dermatoses, par exemple du cuir chevelu) et le prurit généralisé (qui peut avoir des causes environnementales, pathologiques ou psychologiques). D'une manière générale, les origines du prurit restent complexes et mal connues. Des exemples de principes actifs connus dans la littérature pour maîtriser au moins partiellement le prurit sont les anti-inflammatoires tels que les corticostéroïdes, les anti-irritants tels que le camphre, les anesthésiques tels que la pramoxine, les antihistaminiques tels que la cyproheptadine (EP-A-0 104 782), et les anti-éphidrotiques tels que le chlorhydrate d'aluminium (EP-A-0 059 882). En outre, EP-A-0 524 873 divulgue une composition anti-prurigineuse contenant comme principe actif un extrait de racine d'harpagophyton qui est décrit comme ayant une fonction anti-histaminique.

L'invention propose une composition pharmaceutique à base d'Arctium majus, utilisable notamment dans le traitement du prurit, comprenant :

a) de 10 à 25% en poids d'un extrait hydro-glycolique d'Arctium majus ;
b) de 1 à 5% en poids d'un agent complexant ;
c) de 50 à 70% en poids d'un épaississant ;
d) de 5 à 10% en poids d'un solubilisant,

par rapport au poids total de la composition.

Eventuellement, la composition renferme en outre de 10 à 15% en poids d'au moins un parfum.

Selon une forme d'exécution préférée, la composition selon l'invention renferme environ 18% en poids d'un extrait hydro-glycolique d'Arctium majus ; environ 3% en poids d'un agent complexant ; environ 60% en poids d'un épaississant ; environ 7% en poids d'un solubilisant ; et environ 12% en poids d'au moins un parfum.

De préférence, le solubilisant est un mélange d'alkylphénol polyoxyéthyléné et de mono-laurate de sorbitan polyoxyéthyléné ; l'agent complexant est le sel tétrasodique d'éthylène diamine tétraacétique ; et l'épaississant est un diéthanolamide d'acide gras de coprah.

L'invention concerne également une solution lavante à usage pharmaceutique comprenant :

a) d'environ 5 à environ 10% en poids de la composition selon l'invention ;
b) d'environ 20 à environ 25% en poids d'un excipient convenable ;
c) d'environ 1 à environ 5% en poids d'un agent mouillant ; et
d) d'environ 0,1 à environ 5% en poids d'un agent surgraissant,

le reste étant constitué d'eau.

Selon une forme d'exécution préférée, la solution lavante selon l'invention renferme :

a) environ 6,5 % en poids de la composition selon l'invention ;
b) environ 23,5% en poids d'un excipient ;
c) environ 2% en poids d'un agent mouillant ; et
d) environ 1% en poids d'un agent surgraissant.

De préférence, l'excipient est la cocamidopropyl bétaïne ; l'agent mouillant est l'oxyde de stéaramine ; et l'agent surgraissant est constitué d'un mélange de glycérides d'acides caprique et caprylique.

L'invention concerne également l'utilisation de la composition mentionnée ci-dessus pour fabriquer un médicament anti-prurigineux.

Avantageusement, le médicament anti-prurigineux précité est adapté à une application topique. Dans ce cas, il peut s'agir de la solution lavante selon l'invention, qui peut de préférence être utilisée comme solution lavante gynécologique.

L'invention sera mieux comprise à la lumière de l'exemple non limitatif suivant.

Exemple

Préparation du concentré

Afin de préparer une composition selon l'invention, on a tout d'abord dissous complètement, sous forte agitation et à température ambiante, 89,55 kg d'extrait hydro-glycolique de racines d'Arctium majus, disponible auprès de GIFRER BARBEZAT, Decines, France, et 14,925 kg de sel tétrasodique d'EDTA pour obtenir un premier mélange. Dans une cuve séparée, on a réalisé un second mélange homogène de 44,775 kg d'un parfum de base, vendu par V. MANE FILS, Le Bar sur Loup, France sous la référence E PHF145/01, avec 14,935 kg d'une huile essentielle de lavande et 37,315 kg d'un solubilisant, qui était un mélange défini d'alkylphénol polyoxyéthyléné et de mono-laurate de sorbitan polyoxyéthyléné, disponible auprès de GATTEFOSSE, Saint-Priest, France sous la dénomination commerciale Solubilisant GAMMA 2420. Le premier et le second mélanges ont ensuite été successivement ajoutés, sous agitation modérée, à 298,500 kg d'un épaississant, constitué d'un diéthanolamide d'acide gras de coprah vendu par SEPPIC, France sous la dénomination commerciale ORAMIDE DL 215, jusqu'à obtention d'une solution homogène.

Le concentré obtenu avait la composition suivante (les proportions des différents constituants sont exprimées en pourcentages pondéraux par rapport au poids total du concentré) :

| Extrait d'Arctium mains | 17,910 % |
|---|---|
| EDTA tétrasodique | 2,985 % |
| Epaississant | 59,700 % |
| Huile essentielle de lavande | 2,987 % |
| Parfum de base | 8,955 % |
| Solubilisant | 7.463 % |
| Total | 100,000 % |

Préparation de la solution lavante gynécologique

Le concentré précédent a été utilisé pour fabriquer une solution lavante. Le procédé mis en oeuvre a d'abord consisté à introduire lentement, sous agitation lente, 201 kg du concentré obtenu précédemment dans de l'eau purifiée préalablement portée à une température d'environ 70°C, de façon à obtenir un mélange homogène. On a ensuite ajouté à cette solution 54 kg d'un agent mouillant, à savoir l'oxyde de stéaramine disponible sous la dénomination commerciale AMMONYX SO auprès de MILLMASTER-ONYX INTERNATIONAL, INC., Fairfield, New Jersey, USA, puis 705 kg d'un excipient, qui était la cocamidopropyl bétaïne disponible sous la dénomination commerciale TEGO BETAIN L7 auprès de Th. GOLDSCHMIDT AG, Essen, Allemagne, et, enfin, 30 kg de SOFTIGEN 765, qui est un agent surgraissant constitué d'un mélange de glycérides d'acide caprylique et caprique et disponible auprès de HÜLS, Marl, Allemagne. L'agitation lente a été maintenue tandis que la température a été abaissée à 25°C. Le pH a ensuite été ajusté à 7,7 à l'aide d'une solution contenant 1,02 kg d'hydroxyde de sodium. Il est bien entendu que, en fonction des lots de matières premières utilisés, l'ajustage du pH à la valeur indiquée peut se faire en ajoutant à la formulation soit de l'hydroxyde de sodium, comme cela est le cas dans cet exemple, soit de l'acide chlorhydrique. En outre, en fonction de la viscosité voulue pour la formulation, il peut être utile d'ajouter à celle-ci du chlorure de sodium. Dans l'exemple précédent, 36 kg de chlorure de sodium ont été ajoutés au mélange.
La solution lavante obtenue avait la composition suivante (les proportions des différents constituants sont exprimées en pourcentages pondéraux par rapport au poids total de la composition) :

| Excipient | 23,5 % |
|---|---|
| Extrait d'Arctium majus | 1,2 % |
| Chlorure de sodium | 1,2 % |
| EDTA tétrasodique | 0,2 % |
| Huile essentielle de lavande | 0,2 % |
| Parfum de base | 0,6 % |
| Epaississant | 4,0 % |
| Agent surgraissant | 1,0 % |
| Solubilisant | 0,5 % |
| Agent mouillant | 1,8 % |
| Eau purifiée | qsp 100,0 % |

La solution lavante selon l'invention est adaptée à une utilisation comme solution lavante gynécologique dans le traitement du prurit.

Revendications

1. Composition pharmaceutique à base d'Arctium majus, comprenant :

   a) de 10 à 25% en poids d'un extrait hydro-glycolique d'Arctium majus ;
   b) de 1 à 5% en poids d'un agent complexant ;
   c) de 50 à 70% en poids d'un épaississant ;
   d) de 5 à 10% en poids d'un solubilisant,

   par rapport au poids total de la composition.

2. Composition selon la revendication 1, **caractérisée en ce qu'**elle renferme en outre de 10 à 15% en poids d'au moins un parfum.

3. Composition selon la revendication 2, **caractérisée en ce qu'**elle renferme environ 18% en poids d'un extrait hydro-glycolique d'Arctium majus ; environ 3% en poids d'un agent complexant ; environ 60% en poids d'un épaississant ; environ 7% en poids d'un solubilisant ; et environ 12% en poids d'au moins un parfum.

4. Composition selon l'une quelconque des revendications 1 à 3, **caractérisée en ce que** ledit solubilisant est constitué d'un mélange d'alkylphénol polyoxyéthyléné et de mono-laurate de sorbitan polyoxyéthyléné.

5. Composition selon l'une quelconque des revendications 1 à 4, **caractérisée en ce que** ledit agent complexant est le sel tétrasodique d'éthylène diamine tétraacétique.

**6.** Composition selon l'une quelconque des revendications 1 à 5, **caractérisée en ce que** ledit épaississant est un diéthanolamide d'acide gras de coprah.

**7.** Composition selon l'une quelconque des revendications précédentes, pour son utilisation comme médicament.

**8.** Solution lavante à usage pharmaceutique comprenant :

a) d'environ 5 à environ 10% en poids de la composition selon l'une quelconque des revendications précédentes ;
b) d'environ 20 à environ 25% en poids d'un excipient convenable ;
c) d'environ 1 à environ 5% en poids d'un agent mouillant ; et
d) d'environ 0,1 à environ 5% en poids d'un agent surgraissant,

le reste étant constitué d'eau.

**9.** Solution lavante selon la revendication 8, **caractérisée en ce qu'**elle comprend :

a) environ 6,5 % en poids de la composition selon l'une des revendications 1 à 6 ;
b) environ 23,5% en poids d'un excipient ;
c) environ 2% en poids d'un agent mouillant ; et
d) environ 1% en poids d'un agent surgraissant,

le reste étant constitué d'eau.

**10.** Solution lavante selon la revendication 8 ou 9, **caractérisée en ce que** ledit excipient est la cocamidopropyl bétaïne.

**11.** Solution lavante selon l'une quelconque des revendications 8 à 10, **caractérisée en ce que** ledit agent mouillant est l'oxyde de stéaramine.

**12.** Solution lavante selon l'une quelconque des revendications 8 à 11, **caractérisée en ce que** ledit agent surgraissant est constitué d'un mélange de glycérides d'acides caprique et caprylique.

**13.** Solution lavante selon l'une quelconque des revendications 8 à 12, pour son utilisation comme médicament.

**14.** Utilisation de la solution lavante selon l'une quelconque des revendications 8 à 12 pour fabriquer un médicament anti-prurigineux.

**15.** Utilisation d'une composition selon l'une quelconque des revendications 1 à 6 pour fabriquer un médicament anti-prurigineux.

## Claims

**1.** Pharmaceutical composition based on Arctium majus, comprising:

a) from 10 to 25% by weight of an aqueous-glycolic extract of Arctium majus;
b) from 1 to 5% by weight of a complexing agent;
c) from 50 to 70% by weight of a thickener;
d) from 5 to 10% by weight of a solubilizer,

relative to the total weight of the composition.

**2.** Composition according to Claim 1, **characterized in that** it contains, in addition, from 10 to 15% by weight of at least one perfume.

**3.** Composition according to Claim 2, **characterized in that** it contains about 18% by weight of an aqueous-glycolic extract of Arctium majus; about 3% by weight of a complexing agent; about 60% by weight of a thickener; about 7% by weight of a solubilizer; and about 12% by weight of at least one perfume.

**4.** Composition according to any one of Claims 1 to 3, **characterized in that** the said solubilizer consists of a mixture of polyoxyethylenated alkylphenol and polyoxyethylenated sorbitan monolaurate.

**5.** Composition according to any one of Claims 1 to 4, **characterized in that** the said complexing agent is ethylenediaminetetraacetic acid tetrasodium salt.

**6.** Composition according to any one of Claims 1 to 5, **characterized in that** the said thickener is a copra fatty acid diethanolamide.

**7.** Composition according to any one of the preceding claims, for its use as a medicament.

**8.** Washing solution for pharmaceutical use comprising:

a) from about 5 to about 10% by weight of the composition according to any one of the preceding claims;
b) from about 20 to about 25% by weight of a suitable excipient;
c) from about 1 to about 5% by weight of a wetting agent; and
d) from about 0.1 to about 5% by weight of a superfatting agent,

the remainder consisting of water.

9. Washing solution according to Claim 8, **characterized in that** it comprises:

a) about 6.5% by weight of the composition according to one of Claims 1 to 6;
b) about 23.5% by weight of an excipient;
c) about 2% by weight of a wetting agent; and
d) about 1% by weight of a superfatting agent,

the remainder consisting of water.

10. Washing solution according to Claim 8 or 9, **characterized in that** the said excipient is cocamidopropyl betaine.

11. Washing solution according to any one of Claims 8 to 10, **characterized in that** the said wetting agent is stearamine oxide.

12. Washing solution according to any one of Claims 8 to 11, **characterized in that** the said superfatting agent consists of a mixture of capric and caprylic acid glycerides.

13. Washing solution according to any one of Claims 8 to 12, for its use as a medicament.

14. Use of the washing solution according to any one of Claims 8 to 12 to manufacture an antipruritic medicament.

15. Use of a composition according to any one of Claims 1 to 6 to manufacture an antipruritic medicament.


**Patentansprüche**

1. Pharmazeutische Zusammensetzung auf der Grundlage von Arctium majus, bestehend aus:

a) 10 bis 25 Gew.-% eines wässrigen Glykolextrakts von Arctium majus,
b) 1 bis 5 Gew.-% eines Komplexiermittels,
c) 50 bis 70 Gew.-% eines Verdickungsmittels,
d) 5 bis 10 Gew.-% eines Solubilisierungsmittels,

bezogen auf das Gesamtgewicht der Zusammensetzung.

2. Zusammensetzung nach Anspruch 1, **dadurch gekennzeichnet, dass** sie außerdem 10 bis 15 Gew.-% mindestens eines Duftstoffs enthält.

3. Zusammensetzung nach Anspruch 2, **dadurch ge-**

**kennzeichnet, dass** sie etwa 18 Gew.-% eines wässrigen Glykolextrakts von Arctium majus, etwa 3 Gew.-% eines Komplexiermittels, etwa 60 Gew.-% eines Verdickungsmittels, etwa 7 Gew.-% eines Solubilisierungsmittels und etwa 12 Gew.-% mindestens eines Duftstoffs enthält.

4. Zusammensetzung nach einem der Ansprüche 1 bis 3, **dadurch gekennzeichnet, dass** das Solubilisierungsmittel aus einer Mischung von polyoxyethyleniertem Alkylphenol und polyoxyethyleniertem Sorbitanmonolaurat besteht.

5. Zusammensetzung nach einem der Ansprüche 1 bis 4, **dadurch gekennzeichnet, dass** das Komplexiermittel das Tetranatriumsalz der Ethylendiamintetraessigsäure ist.

6. Zusammensetzung nach einem der Ansprüche 1 bis 5, **dadurch gekennzeichnet, dass** das Verdikkungsmittel ein Koprafettsäurediethanolamid ist.

7. Zusammensetzung nach einem der vorhergehenden Ansprüche bei ihrer Verwendung als Arzneimittel.

8. Waschlösung zu pharmazeutischen Zwecken, enthaltend:

a) etwa 5 bis etwa 10 Gew.-% der Zusammensetzung nach einem der vorhergehenden Ansprüche,
b) etwa 20 bis etwa 25 Gew.-% einer geeigneten Grundsubstanz,
c) etwa 1 bis etwa 5 Gew.-% eines Netzmittels und
d) etwa 0,1 bis etwa 5 Gew.-% eines Rückfettungsmittels,

wobei der Rest aus Wasser besteht.

9. Waschlösung nach Anspruch 8, **dadurch gekennzeichnet, dass** sie enthält:

a) etwa 6,5 Gew.-% der Zusammensetzung nach einem der Ansprüche 1 bis 6,
b) etwa 23,5 Gew.-% einer Grundsubstanz,
c) etwa 2 Gew.-% eines Netzmittels und
d) etwa 1 Gew.-% eines Rückfettungsmittels,

wobei der Rest aus Wasser besteht.

10. Waschlösung nach Anspruch 8 oder 9, **dadurch gekennzeichnet, dass** die Grundsubstanz Cocamidopropylbetain ist.

11. Waschlösung nach einem der Ansprüche 8 bis 10, **dadurch gekennzeichnet, dass** das Netzmittel Stearaminoxid ist.

**12.** Waschlösung nach einem der Ansprüche 8 bis 11, **dadurch gekennzeichnet, dass** das Rückfettungsmittel aus einer Mischung von Kaprin- und Kaprylsäureglyceriden besteht.

**13.** Waschlösung nach einem der Ansprüche 8 bis 12 bei ihrer Verwendung als Arzneimittel.

**14.** Verwendung der Waschlösung nach einem der Ansprüche 8 bis 12 zur Herstellung eines Arzneimittels gegen Juckreiz.

**15.** Verwendung einer Zusammensetzung nach einem der Ansprüche 1 bis 6 zur Herstellung eines Arzneimittels gegen Juckreiz.